(19)

Europäisches
Patentamt

European
Patent Office

Office européen
des brevets

(11)     **EP 1 589 067 B1**

(12)                              **EUROPEAN PATENT SPECIFICATION**

(45) Date of publication and mention
     of the grant of the patent:
     **25.06.2008 Bulletin 2008/26**

(51) Int Cl.:
     *C08L 3/04* (2006.01)          *A61K 47/36* (2006.01)
     *A61K 9/00* (2006.01)          *B65D 81/34* (2006.01)

(21) Application number: **05007549.8**

(22) Date of filing: **06.04.2005**

(54) **Edible film**

Essbare Folie

Film comestible

(84) Designated Contracting States:
     **DE FR GB**

(30) Priority: **20.04.2004 JP 2004124107**

(43) Date of publication of application:
     **26.10.2005 Bulletin 2005/43**

(73) Proprietor: **MATSUTANI CHEMICAL INDUSTRY
     CO., LTD.
     Itami-shi,
     Hyogo-ken (JP)**

(72) Inventors:
     • **Kitamura, Kosei
       Takarazuka-shi
       Hyogo-ken (JP)**

• **Yanetani, Yoshifumi
  Nara-shi
  Nara-ken (JP)**
• **Kataoka, Hiroyuki
  Takarazuka-shi
  Hyogo-ken (JP)**
• **Okuma, Kazuhiro
  Sanda-shi
  Hyogo-ken (JP)**

(74) Representative: **Müller-Boré & Partner
     Patentanwälte
     Grafinger Strasse 2
     81671 München (DE)**

(56) References cited:
     **EP-A- 0 547 551          US-A- 3 527 646
     US-A1- 2003 099 692        US-B1- 6 375 981
     US-B2- 6 656 493**

**Description**

The present invention relates to an edible film.

Edible films are dissolved by saliva in the oral cavity. Such films can be used as packages or carriers of foodstuffs, vitamins, pharmaceuticals. They can also be used as flavor films when provided with flavors, thus attracting attention as a new form of food product for refreshment, prevention of bad breath.

In general, edible films are manufactured by dissolving a film-forming agent such as starch, a gelling agent, a plasticizer, in water, drying the mixture in film shape, cutting the resulting film to a suitable size, and placing it in a container.

Edible films are required to decompose easily in the oral cavity, i.e. to have excellent dissolvability in the mouth. They are also required to be free from cracking or curling when dried, i.e. to have excellent drying resistance. Furthermore, they are required to have excellent resistance to blocking, which is caused by the adhesion of films when they absorb moisture in air or melt with heat.

In a film such as a wafer for wrapping, a native starch has been used as the main component. Because of its high-viscosity, the concentration of such starch must be lowered for film preparation, resulting in a thin film. This thin film readily absorbs moisture, causing blocking. A film containing a protein such as gelatin as its main component tends to absorb moisture or melt under the conditions of high temperature and high humidity, thus easily causing blocking, especially in summer.

Japanese Unexamined Patent Publication No. 1993-236885 discloses a film using pullulan and carrageenan to increase drying resistance. This film, however, has low resistance to moisture, causing blocking under high humidity conditions.

U.S. Patent No. 6656493 discloses a film comprising sodium alginate and maltodextrin. A low-molecular-weight maltodextrin is added to this film to improve dissolvability in the mouth. However, if the concentration of the maltodextrin is raised, the moisture resistance of the film is lessened, easily causing blocking. Furthermore, the drying resistance of the film is not satisfactory.

U.S. Patent No. 6528088 discloses a film comprising a modified starch, such as etherified starch or esterified starch, in combination with a plasticizer to improve the plasticity of the film, wherein the weight ratio of the plasticizer to the starch is at least 0.5:1. However, because of the large amount of plasticizer, the film has an unpleasant mouth feel and poor blocking resistance.

U.S. Patent Application Publication No. 2003/0099692 discloses a film comprising a modified starch as a main component, wherein usable starches are chemically modified starches such as those which have been crosslinked, acetylated and organically esterified, hydroxyethylated and hydroxypropylated, etc.; and conversion products derived from starches by oxidation, enzymatic conversion, acid hydrolysis, etc. In this film, however, a modified starch is substantially used alone, so that the blocking resistance of this film is insufficient.

US-patent 3,527,646 describes a method of coating foods with an edible amorphous film containing a pregelatinized starch as the essential ingredient. EP-A-0 547 551 describes compositions useful as edible films comprising, by weight, 5 to 40% modified starch, 5 to 40% gelatin, 10 to 40% plasticizer, 5 to 40% water, and, optionally, 5 to 40% lipid, wherein the compositions have a viscosity of less than about 370,000 cps at 80°C and the edible films are effective to provide water, lipid, solute, gas, physical or microbial barriers in foods. US-patent 6,375,981 describes film-forming compositions that can comprise, on a dry solid basis, 25 to 75 percent by weight of certain starch derivatives having a DE less than about 1, 25 to 75% plasticizer, and 0.1 to 15% hydrocolloid gum.

An object of the present invention is to provide an edible film having excellent properties in terms of dissolvability, drying resistance, and blocking resistance.

To achieve this object, the present inventors conducted extensive research. As a result, the inventors found that the above object can be achieved by an edible film comprising a mixture of at least one modified starch selected from etherified high amylose starches, and at least one modified starch selected from etherified starch oxidation products; a gelling agent; and a plasticizer. Based on this finding, the inventors have accomplished the present invention.

The present invention relates to the following edible films.

    1. An edible film comprising:

        (A) a modified starch mixture of (a) at least one modified starch selected from the group consisting of etherified high amylose starches and esterified high amylose starches, and (b) at least one modified starch selected from the group consisting of etherified starch oxidation products, etherified starch acid decomposition products, and etherified starch enzymatic decomposition products;
        (B) at least one gelling agent; and
        (C) at least one plasticizer;

    wherein the proportion of (a)/(b) in the modified starch mixture (A) is in the range of (a) 30% to 90% by weight/(b) 70% to 10% by weight, based on the total weight of (a) and (b).

2. An edible film according to item 1, wherein the proportion of (a)/(b) in the modified starch mixture (A) is in the range of (a) 40% to 80% by weight/(b) 60% to 20% by weight, based on the total weight of (a) and (b).

3. An edible film according to item 1, wherein the proportion of gelling agent (B) is in the range of 12 to 50 parts by weight per 100 parts by weight of the modified starch mixture (A).

4. An edible film according to item 1, wherein the proportion of plasticizer (C) is in the range of 3 to 40 parts by weight per 100 parts by weight of the modified starch mixture (A).

5. An edible film according to item 1, wherein the etherified high amylose starch is hydroxypropylated high amylose corn starch.

6. An edible film according to item 1, wherein the esterified high amylose starch is acetylated high amylose corn starch.

7. An edible film according to item 1, wherein the etherified starch oxidation product is a hydroxypropylated starch oxidation product.

8. An edible film according to item 1, wherein the etherified starch acid decomposition product is a hydroxypropylated starch acid decomposition product.

9. An edible film according to item 1, wherein the etherified starch enzymatic decomposition product is a hydroxypropylated starch amylase decomposition product.

10. An edible film according to item 1, wherein the film has a thickness of 20 to 200 $\mu$m.

The present invention provides an edible film comprising (A) a modified starch mixture of (a) at least one modified starch selected from the group consisting of etherified high amylose starches and esterified high amylose starches with (b) at least one modified starch selected from the group consisting of etherified starch oxidation products, etherified starch acid decomposition products, and etherified starch enzymatic decomposition products; (B) gelling agent (s); and (C) plasticizer(s).

(A) Modified starch mixture

Modified starch mixture (A) functions as a film-forming agent. In the present invention, it is essential to use as modified starch mixture (A), a mixture of (a) at least one modified starch selected from the group consisting of etherified high amylose starches and esterified high amylose starches with (b) at least one modified starch selected from the group consisting of etherified starch oxidation products, etherified starch acid decomposition products, and etherified starch enzymatic decomposition products.

The term "high amylose starch" is used herein to mean a starch containing at least 30 wt.% amylose, and preferably 40 to 80 wt.% amylose.

In this specification, "wt.%" means percent by weight.

Examples of high amylose starches include high amylose corn starch, mung bean starch, yellow pea starch.

High amylose starches, which have a linear chain structure similar to that of cellulose, exhibit excellent film-formimg properties but retrograde rapidly. Using etherified high amylose starches and esterified high amylose starches as component (a) of modified starch mixture (A) provides a stable starch solution since high amylose starches that have been etherified or esterified are capable of maintaining excellent film-forming properties while resisting retrogradation.

Preferable examples of etherified high amylose starches include hydroxypropylated high amylose corn starch. Preferable examples of esterified high amylose starches include acetylated high amylose corn starch.

Hydroxypropylated high amylose corn starch can be produced by, for example, reacting a high amylose corn starch with propylene oxide. Acetylated high amylose corn starch can be produced by, for example, reacting a high amylose corn starch with acetic anhydride under dilute alkaline conditions.

The DS (Degree of Substitution) of the hydroxypropylated high amylose starches and acetylated high amylose starches is preferably 0.02 to 0.4.

This degree of substitution expresses the molar equivalents of the substituted hydroxyl groups per glucose unit in a starch or decomposition product thereof. The degree of hydroxypropyl substitution can be determined according to the method of Johnson (Analytical Chemistry, Vol.41, No.6, 859-860, 1969). The degree of acetyl substitution can be determined according to the method of Smith (Starch: Chemistry and Technology, Vol.2, 610-612, 1967, Academic Press).

The viscosity of a 20 wt.% aqueous solution of hydroxypropylated high amylose starch or acetylated high amylose starch as component (a) is preferably in the range of 30,000 to 400,000 mPa·s (50°C, B type viscometer, 10 rpm). When the viscosity is below this range, the drying resistance of a film tends to be low, and when the viscosity is above this range, the viscosity at the time of film preparation tends to be too high, resulting in poor workability. The above viscosity of component (a) is more preferably in the range of 50,000 to 300,000 mPa·s.

Etherified starch oxidation products, etherified starch acid decomposition products, and etherified starch enzymatic decomposition products as component (b) of modified starch mixture (A) improve the dissolvability of films in the mouth, which cannot be satisfactorily obtained by the use of component (a) alone. They also improve the clarity, surface smoothness, gloss, of films; and enhance film-forming properties.

Preferable etherified starch oxidation products are, for example, oxidation products of hydroxypropylated starches. Preferable starch materials thereof are, for example, tapioca starch, potato starch, corn starch, wheat starch, waxy corn starch. Especially preferable etherified starch oxidation products are hydroxypropylated tapioca starch oxidation product, hydroxypropylated potato starch oxidation product, and hydroxypropylated waxy corn starch oxidation product.

An etherified starch oxidation product can be prepared by, for example, adding an 2 to 20 wt.% aqueous solution of sodium hypochlorite dropwise to an 35 to 45 wt.% aqueous slurry of etherified starch with stirring at room temperature for 30 to 150 minutes to oxidize or bleach the starch.

Preferable etherified starch acid decomposition products are, for example, acid decomposition products of hydroxypropylated starches. Preferable starch materials thereof are, for example, tapioca starch, potato starch, corn starch, wheat starch, waxy corn starch. Especially preferable etherified starch acid decomposition products are acid decomposition products of hydroxypropylated tapioca starch, hydroxypropylated potato starch, and hydroxypropylated waxy corn starch.

Etherified starch acid decomposition products can be prepared by methods such as acid immersion, acid roasting. In the case of acid immersion, an acid decomposition product can be prepared by, for example, immersing an etherified starch in an 0.5 to 10 wt.% aqueous solution of inorganic acid such as hydrochloric acid or sulfuric acid with stirring at about 50°C for 30 minutes to 5 hours. In the case of acid roasting, an acid decomposition product can be prepared by, for example, adding an 0.1 to 1 wt.% aqueous solution of inorganic acid such as hydrochloric acid or nitric acid to an etherified starch in an amount of about 10% by weight, followed by hot-air drying and then roasting at 120°C to 220°C for 30 to 120 minutes.

Preferable etherified starch enzymatic decomposition products are, for example, amylase decomposition products of hydroxypropylated starches. Preferable starch materials thereof are, for example, tapioca starch, potato starch, corn starch, wheat starch, waxy corn starch. Especially preferable etherified starch enzymatic decomposition products are amylase decomposition products of hydroxypropylated tapioca starch, hydroxypropylated potato starch, and hydroxypropylated waxy corn starch.

Etherified starch enzymatic decomposition products can be prepared by, for example, heat-resistant α-amylase treatment.

The viscosity of a 20 wt% aqueous solutions of an etherified starch oxidation product, etherified starch acid decomposition product, or etherified starch enzymatic decomposition product as component (b) is preferably not more than 5,000 mPa·s (50°C, B type viscometer, 10 rpm). When the viscosity is above 5,000 mPa·s, the dissolvability of the film in the mouth tends to be poor. The above viscosity of component (b) is more preferably in the range of 10 to 3,000 mPa·s.

DE is a numerical value showing the degree of decomposition of a starch decomposition product formed by hydrolyzing starch with an enzyme or acid, and is expressed by the following formula:

$$DE = [(direct\ reducing\ sugar)/(solids\ content)] \times 100$$

Analysis of direct reducing sugar can be performed by the Willstatter-Schudel method.

The proportion of (a)/(b) in the modified starch mixture (A) is in the range of (a) 30 to 90 wt.%/(b) 70 to 10 wt.%, based on the total weight of (a) and (b). When the amount of component (a) is below this range, the blocking resistance of the film tends to be low, and when the amount thereof is above this range, the dissolvability of the film in the mouth tends to be poor. The proportion of (a)/(b) is preferably in the range of (a) 40 to 80 wt.%/ (b) 60 to 20 wt.%, and more preferably in the range of (a) 40 to 75 wt.%/(b) 60 to 25 wt.%, based on the total weight of (a) and (b).

(B) Gelling agent

Gelling agents used for the film of the present invention have the property of gelling when cooled. Examples thereof include carrageenan, gelatin, gellan gum, agar, alginates, and these may be used singly or in combination of two or more. Examples of alginates include alkali metal alginates such as sodium alginate; alkaline earth metal alginates such as calcium alginate.

The proportion of gelling agent (B) is preferably in the range of 12 to 50 parts by weight per 100 parts by weight of the modified starch mixture (A). When the proportion of gelling agent (B) is below this range, the film tends to readily curl; and when the proportion thereof is above this range, the dissolvability of the film in the mouth tends to be low. The proportion of gelling agent (B) is more preferably in the range of 15 to 30 parts by weight per 100 parts by weight of the modified starch mixture (A).

(C) Plasticizer

Plasticizers used for the film of the present invention have the property of softening the film. Examples thereof include glycerin, sugar alcohols, monosaccharides, oligosaccharides; and these may be used singly or in combination of two or more.

The proportion of plasticizer (C) is preferably in the range of 3 to 40 parts by weight per 100 parts by weight of the modified starch mixture (A). When the proportion of plasticizer (C) is below this range, the drying resistance of the film tends to be diminished; and when the proportion thereof is above this range, the blocking resistance of the film tends to be poor. The proportion of plasticizer (C) is more preferably in the range of 7 to 35 parts by weight per 100 parts by weight of the modified starch mixture (A).

Optional components

In addition to the essential components, i.e. modified starch mixture (A), gelling agent (B) and plasticizer (C), the edible film of the present invention may contain optional components such as nutrients, plant extracts, herbal components, vitamins, minerals, antibacterial agents, anti-inflammatory agents, anti-cariogenic agents, anti-allergic agents, antitussive agents, motion sickness preventives, nitroglycerin, catechin, polyphenols, enzymes, flavors, dyes, emulsifiers, seasonings, fragrances, colorants, fats and oils in a suitable manner depending on the use of the film.

Preparation of edible film

The edible film of the present invention comprises modified starch mixture (A), gelling agent (B), plasticizer (C), and, if necessary, optional components. It may be formed in a film shape in the usual manner. For example, the edible film of the invention can be easily prepared according to the steps of (1) preparing a starting liquid, (2) coating, (3) drying, (4) aging, and (5) cutting. These steps are described below.

(1) Preparation of starting liquid: Components (a) and (b) of modified starch mixture (A) are dispersed in water at room temperature in an amount of 1.5 to 4.5 times the total amount of these components. The resulting mixture is usually heated to 80°C to 95°C to gelatinize the starch. The liquid is maintained at 60°C or higher while a gelling agent (B) and a plasticizer (C) are added thereto, mixed and dissolved. If necessary, optional components are dissolved or dispersed in the liquid, thus giving a starting liquid.

(2) Coating: The starting liquid is maintained at 50°C to 55°C while the solids concentration thereof (usually 20 to 30 wt.%) is adjusted such that the viscosity of the liquid is 2,000 to 5,000 mPa·s. The liquid is then spread, using an applicator to coat a synthetic resin sheet, within a suitable coating film thickness range. The synthetic resin sheet may be, for example, a polyester-based resin sheet.

(3) Drying: The resulting film is dried by hot air of 80°C to 110°C. To obtain excellent properties in terms of drying resistance, mouth feel, dissolvability in the mouth, the dry film preferably has a thickness of 20 to 200 $\mu$m. To obtain excellent properties in terms of feel in the mouth, preservability, the film preferably has a water content of 5% to 15% by weight.

(4) Aging: The film is usually left for 24 hours in a humidity-adjusted room with a temperature of 25°C and a relative humidity of 45% to stabilize its water content.

(5) Cutting: The aged film is removed from the synthetic resin sheet and cut in a suitable manner to manufacture film products.

The edible film of the present invention provides the following remarkable effects.

(i) Although not easily dissolving in water of about room temperature, the edible film of the present invention is decomposed by the action of salivary amylase in the oral cavity, being rapidly dissolved. This is because the main component of the film is a specific modified starch mixture, which is hydrolyzed by amylase in the oral cavity, thus easily disintegrating or dissolving the film. The film of the invention therefore has excellent dissolvability in the mouth.

(ii) The edible film of the invention does not easily crack or curl when dried. The film therefore has excellent drying resistance.

(iii) The edible film of the invention does not undergo blocking, which is caused by the adhesion of films that absorb moisture in air or melt with heat. Having excellent properties in terms of moisture resistance and heat resistance, the film is resistant to blocking even under high humidity and high temperature conditions, especially in summer. The film of the invention therefore has remarkably excellent blocking resistance.

(iv) The excellent drying resistance and blocking resistance of the edible film of the invention facilitates its adaptation to the environment. Because of such properties, for example, a container used to store or carry the edible film does not require rigorous sealing.

(v) The edible film of the present invention can be suitably used as a package for foodstuffs such as dietary supplements, pharmaceuticals, and as a carrier for very small quantities of effective components such as pharmaceuticals, food extracts. It is also suitable as a flavor film or a mouth-freshening film, when provided with emulsified fat-soluble flavors, emulsified fats and oils.

The film of the invention, when used for packaging foodstuffs, is suitable as a film for packages of, for example, oil-containing components such as powdered soup and chili oil; ingredients such as spices and ramen noodles.

Furthermore, the film of the invention is useful not only for healthy people but also for those who have difficulties swallowing when taking vitamins, pharmaceuticals.

EXAMPLES

The present invention is described below in further detail with reference to examples. The films in the examples were prepared according to the above-mentioned method, with a length of 3.3 cm and a width of 2.3 cm. The films were tested with respect to their film-forming properties, amylase decomposability, dissolvability in the mouth, drying resistance, and blocking resistance, using the following methods.

Film-forming properties: Films formed in the coating step were examined for surface unevenness such as concavity and convexity before cutting, and then examined for their surface conditions and thickness uniformity after cutting. The film-forming properties were evaluated according to the following criteria.

A: The film before cutting was free from surface unevenness, and the film after cutting had a smooth surface and uniform thickness.

B: The surface of the film before cutting was partly uneven, and the film after cutting had a partly uneven surface and a somewhat non-uniform thickness.

C: The surface of the film before cutting was wholly uneven, and the film after cutting had a wholly uneven surface and non-uniform thickness.

Amylase decomposability: 100 $\mu$L of 3 mg of human-derived salivary amylase ("Type IX-A", product of Sigma) /10 mL (0.45 mM Bis-Tris buffer solution, containing 0.9 mM $CaCl_2$, pH 6.0) was dropped onto a film on a net. The time taken until a drop fell through the net was measured to evaluate the amylase decomposability of the film according to the following criteria.

A: The time taken until a drop fell through was less than 2 minutes; therefore, decomposition was rapid.

B: The time taken until a drop fell through was 2 to less than 10 minutes; therefore, decomposition was somewhat slow.

C: The time taken until a drop fell through was at least 10 minutes; therefore, decomposition was slow.

Dissolvability in the mouth: Six adult males placed a film in their mouths. The dissolvability of the film in the mouth was evaluated according to the following criteria.

A: The film dissolved in less than 20 seconds without leaving any lumps or masses (so-called "dama") in the mouth.

B: Although leaving no lumps or masses in the mouth, the film took at least 20 seconds to dissolve.

C: The film did not completely dissolve in the mouth, leaving lumps or masses.

Drying resistance: Films were stored for 1 week in a desiccator adjusted to RH 20%, 25°C. The cracking and curling of the films were evaluated according to the following criteria. Cracking A: The film did not crack when bended or when twisted.

B: The film did not crack when bended, but cracked when twisted.

C: The film cracked when a load was applied thereto. Curling

A: The film was completely flat.

B: The film had a natural curl.

C: The film was curled as if shrunken.

Blocking resistance: Films were stored for 24 hours in a desiccator adjusted to relative humidity (RH): 45%, temperature: 50°C (high temperature conditions) or stored for 1 week in a desiccator adjusted to RH 81%, 25°C (high humidity conditions). The blocking resistance of the films was evaluated according to the following criteria.

A: Blocking did not occur. No changes occurred.

B: Although slight blocking occurred, it was possible to detach the films from one another.

C: Blocking occurred, and the films could not be detached from one another.

Example 1

Hydroxypropylated high amylose corn starch (DS: 0.12, amylose content: 65 wt.%, viscosity of 20 wt.% aqueous solution (50°C, B type viscometer, 10 rpm): 248,000 mPa·s), an etherified starch oxidation product (viscosity of 20 wt. % aqueous solution (50°C, B type viscometer, 10 rpm): 600 mPa·s) obtained from hydroxypropylated tapioca starch (DS: 0.10) using sodium hypochlorite, κ-carrageenan (product of CP Kelco), and glycerin were used in the ratios shown in Table 1. Five kinds of wrapping films for foodstuffs were prepared with a water content of 11-13 wt.% and a thickness of 60 μm, and subjected to performance tests.

The amounts of the components and test results are shown in Table 1.

Table 1

| | Film No. | 1 | 2 | 3 | 4 | 5 | 6 |
|---|---|---|---|---|---|---|---|
| Component | Hydroxypropylated high amylose corn starch | 0 | 40 | 60 | 70 | 80 | 100 |
| | Hydroxypropylated tapioca starch oxidation product | 100 | 60 | 40 | 30 | 20 | 0 |
| | κ-carrageenan | 20 | 20 | 20 | 20 | 20 | 20 |
| | Glycerin | 13 | 13 | 13 | 13 | 13 | 13 |
| Performance Test | Film-forming properties | A | A | A | A | A | B |
| | Amylase decomposability | A | A | B | B | B | C |
| | Dissolvability in the mouth | A | A | A | A | B | B |
| | Drying resistance (Cracking) | B | B | B | A | A | A |
| | Drying resistance (Curling) | B | A | A | A | B | B |
| | Blocking resistance (high temperature conditions) | A | A | A | A | A | A |
| | Blocking resistance (high humidity conditions) | C | B | A | A | A | A |

As shown in Table 1, films No. 2 to No. 4 of the present invention gave excellent test results. Film No. 5 of the invention also gave generally excellent test results.

In contrast, when hydroxypropylated tapioca starch oxidation product was used alone as a modified starch in film No. 1, blocking resistance under high humidity conditions was impaired. When hydroxypropylated high amylose corn starch was used alone in film No. 6, amylase decomposability was impaired, resulting in poor dissolvability in the mouth. In both cases, the obtained film was unsuitable as a product.

Example 2 (Not according to the present invention)

Acetylated high amylose corn starch (amylose content: 60 wt.%, DS: 0. 05, viscosity of 20 wt.% aqueous solution (50°C, B type viscometer, 10 rpm): 88,000 mPa·s), maltodextrin (trade name: "Pinedex #100", product of Matsutani Chemical Industry Co., Ltd., DE: 3), κ-carrageenan (product of CP Kelco), glycerin, vinca minor extract (plant extract, product of Covex), and flavoring were used in the ratios shown in Table 2. Four kinds of films for dietary supplements were prepared with a water content of 11-13 wt.% and a thickness of 50 μm, and subjected to performance tests.

The amounts of the components and test results are shown in Table 2.

Table 2

| Film No. | | 1 | 2 | 3 | 4 | 5 |
|---|---|---|---|---|---|---|
| Component | Acetylated high amylose corn starch | 0 | 40 | 60 | 70 | 90 |
| | Maltodextrin (DE: 3) | 100 | 60 | 40 | 30 | 10 |
| | κ-carrageenan | 20 | 20 | 20 | 20 | 20 |
| | Glycerin | 10.6 | 10.6 | 10.6 | 10.6 | 10.6 |
| | Vinca minor extract | 2.7 | 2.7 | 2.7 | 2.7 | 2.7 |
| | Flavoring | s.a.* | s.a.* | s.a.* | s.a.* | s.a.* |
| Performance Test | Film-forming properties | C | A | A | A | A |
| | Amylase decomposability | A | A | B | B | B |
| | Dissolvability in the mouth | A | A | A | A | B |
| | Drying resistance (Cracking) | C | B | B | A | A |
| | Drying resistance (Curling) | C | B | A | A | B |
| | Blocking resistance (high temperature conditions) | C | A | A | A | A |
| | Blocking resistance (high humidity conditions) | C | B | B | A | A |

Note (*): "s.a." means suitable amount

As shown in Table 2, when maltodextrin was used alone as a modified starch in film No. 1, the film-forming properties were impaired, and the resulting film had very poor properties with respect to drying resistance and blocking resistance.

In contrast, films No. 2 to No. 4 of the present invention had remarkably excellent drying resistance and blocking resistance since maltodextrin was used in combination with acetylated high amylose corn starch. Film No. 5 of the invention gave generally excellent test results.

Example 3

Hydroxypropylated high amylose corn starch (DS: 0.12, amylose content: 70 wt.%, viscosity of 20 wt.% aqueous solution (50°C, B type viscometer, 10 rpm): 144,000 mPa·s), an amylase decomposition product (trade name: "Foodtex", product of Matsutani Chemical Industry Co., Ltd., viscosity of 20 wt.% aqueous solution (50°C, B type viscometer, 10 rpm): 70 mPa·s) of hydroxypropylated tapioca starch (DS: 0.10), κ-carrageenan (product of CP Kelco), glycerin, an emulsified flavoring (obtained by emulsifying peppermint oil and a sucrose fatty acid ester), sweetener, and coloring matter (blue No. 1) were used in the ratios shown in Table 3. Four kinds of flavor films were prepared with a water content of 11-13 wt.% and a thickness of 45 μm, and subjected to performance tests.

The amounts of the components and test results are shown in Table 3.

8

## Table 3

| Film No. | | 1 | 2 | 3 | 4 |
|---|---|---|---|---|---|
| Component | Hydroxypropylated high amylose corn starch | 0 | 50 | 67.7 | 100 |
| | Amylase decomposition product of hydroxypropylated tapioca starch | 100 | 50 | 32.3 | 0 |
| | κ-carrageenan | 24.2 | 24.2 | 24.2 | 18.5 |
| | Glycerin | 16.1 | 16.1 | 16.1 | 15.3 |
| | Emulsified flavoring | 19.3 | 19.3 | 19.3 | 18.5 |
| | Sweetener | s.a.* | s.a.* | s.a.* | s.a.* |
| | Coloring matter (blue No. 1) | s.a.* | s.a.* | s.a.* | s.a.* |
| Performance Test | Film-forming properties | A | A | A | B |
| | Amylase decomposability | A | A | B | C |
| | Dissolvability in the mouth | A | A | A | B |
| | Drying resistance (Cracking) | B | B | A | A |
| | Drying resistance (Curling) | B | B | A | A |
| | Blocking resistance (high temperature conditions) | A | A | A | A |
| | Blocking resistance (high humidity conditions) | C | B | A | A |

Note (*): "s.a." means suitable amount

As shown in Table 3, films No. 2 and No. 3 of the present invention gave excellent test results.

In contrast, when an amylase decomposition product of hydroxypropylated tapioca starch was used alone as a modified starch in film No. 1, blocking resistance under high humidity conditions was greatly impaired. When hydroxypropylated high amylose corn starch was used alone as a modified starch in film No. 4, amylase decomposability was impaired, resulting in low dissolvability in the mouth. In both cases, the obtained film was unsuitable as a product.

Example 4

Hydroxypropylated high amylose corn starch (DS: 0.12, amylose content: 70 wt.%, viscosity of 20 wt.% aqueous solution (50°C, B type viscometer, 10 rpm): 144,000 mPa·s), an etherified starch oxidation product (viscosity of 20 wt.% aqueous solution (50°C, B type viscometer, 10 rpm): 600 mPa·s) obtained from hydroxypropylated tapioca starch (DS: 0.10) using sodium hypochlorite, κ-carrageenan (product of CP Kelco), glycerin, vitamins, and flavoring were used in the ratios shown in Table 4. Three kinds of vitamin-containing flavor films were prepared with a water content of 7-11 wt.% and a thickness of 50 μm, and subjected to performance tests.

The amounts of the components and test results are shown in Table 4.

Table 4

| Film No. | | 1 | 2 | 3 |
|---|---|---|---|---|
| Component | Hydroxypropylated high amylose corn starch | 50 | 50 | 50 |
| | Hydroxypropylated tapioca starch oxidation product | 50 | 50 | 50 |
| | κ-carrageenan | 7 | 20 | 60 |
| | Glycerin | 9.1 | 8.1 | 6.1 |
| | Vitamin C (L-ascorbic acid) | 7.9 | 7.1 | 5.3 |
| | Vitamin E | 1.1 | 1.0 | 0.8 |
| | Nicotinamide | 1.1 | 1.0 | 0.8 |
| | Flavoring | s.a.* | s.a.* | s.a.* |
| Performance Test | Film-forming properties | A | A | C |
| | Amylase decomposability | A | A | C |
| | Dissolvability in the mouth | A | A | C |
| | Drying resistance (Cracking) | B | B | C |
| | Drying resistance (Curling) | C | A | A |
| | Blocking resistance (high temperature conditions) | A | A | A |
| | Blocking resistance (high humidity conditions) | B | B | A |

Note (*): "s.a." means suitable amount

As shown in Table 4, film No. 2 of the present invention gave excellent test results.

In contrast, in film No. 1, where the amount of gelling agent was decreased, curling of the film increased. In film No. 3, where the amount of gelling agent was increased, the resulting film had poor properties in terms of dissolvability in the mouth.

**Claims**

1. An edible film comprising:

   (A) a modified starch mixture of (a) at least one modified starch selected from the group consisting of etherified high amylose starches and esterified high amylose starches, and (b) at least one modified starch selected from the group consisting of etherified starch oxidation products, etherified starch acid decomposition products, and etherified starch enzymatic decomposition products, wherein the proportion of (a)/(b) in the modified starch mixture (A) is in the range of (a) 30% to 90% by weight/(b) 70% to 10% by weight, based on the total weight of (a) and (b);
   (B) at least one gelling agent; and
   (C) at least one plasticizer.

2. An edible film according to claim 1, wherein the proportion of (a)/(b) in the modified starch mixture (A) is in the range of (a) 40% to 80% by weight/(b) 60% to 20% by weight, based on the total weight of (a) and (b).

3. An edible film according to claim 1 or 2, wherein the proportion of gelling agent (B) is in the range of 12 to 50 parts by weight per 100 parts by weight of the modified starch mixture (A).

4. An edible film according to any one of claims 1 to 3, wherein the proportion of plasticizer (C) is in the range of 3 to 40 parts by weight per 100 parts by weight of the modified starch mixture (A).

5. An edible film according to any one of claims 1 to 4, wherein the etherified high amylose starch is hydroxypropylated high amylose corn starch.

6.  An edible film according to any one of claims 1 to 5, wherein the esterified high amylose starch is acetylated high amylose corn starch.

7.  An edible film according to any one of claims 1 to 6, wherein the etherified starch oxidation product is a hydroxypropylated starch oxidation product.

8.  An edible film according to any one of claims 1 to 7, wherein the etherified starch acid decomposition product is a hydroxypropylated starch acid decomposition product.

9.  An edible film according to any one of claims 1 to 8, wherein the etherified starch enzymatic decomposition product is a hydroxypropylated starch amylase decomposition product.

10. An edible film according to any one of claims 1 to 9, wherein the film has a thickness of 20 to 200 $\mu$m.

**Patentansprüche**

1.  Eßbare Folie, umfassend:

    (A) ein modifiziertes Stärkegemisch aus (a) mindestens einer modifizierten Stärke, ausgewählt aus der Gruppe, bestehend aus veretherten Stärken mit hohem Amylosegehalt und veresterten Stärken mit hohem Amylosegehalt, und (b) mindestens einer modifizierte Stärke, ausgewählt aus der Gruppe, bestehend aus Oxidationsprodukten von veretherter Stärke, Säurezersetzungsprodukten von veretherter Stärke und enzymatischen Zersetzungsprodukten von veretherter Stärke, wobei das Verhältnis von (a)/(b) in dem modifizierten Stärkegemisch (A) in dem Bereich von (a) 30 bis 90 Gew.-%/(b) 70 bis 10 Gew.-%, bezogen auf das Gesamtgewicht von (a) und (b), liegt,
    (B) mindestens ein Geliermittel, und
    (C) mindestens einen Weichmacher.

2.  Eßbarer Film nach Anspruch 1, wobei das Verhältnis von (a)/(b) in dem modifizierten Stärkegemisch (A) in dem Bereich von (a) 40 bis 80 Gew.-%/(b) 60 bis 20 Gew.-%, bezogen auf das Gesamtgewicht von (a) und (b), liegt.

3.  Eßbare Folie nach Anspruch 1 oder 2, wobei das Verhältnis des Geliermittels (B) in dem Bereich von 12 bis 50 Gew.-Teilen, bezogen auf 100 Gew.-Teile des modifizierten Stärkegemisches (A), liegt.

4.  Eßbare Folie nach einem der Ansprüche 1 bis 3, wobei das Verhältnis des Weichmachers (C) in dem Bereich von 3 bis 40 Gew.-Teilen, bezogen auf 100 Gew.-Teile des modifizierten Stärkegemisches (A), liegt.

5.  Eßbare Folie nach einem der Ansprüche 1 bis 4, wobei die veretherte Stärke mit hohem Amylosegehalt hydroxypropylierte Amylomaisstärke bzw. hydroxypropylierte Maisstärke mit hohem Amylosegehalt ist.

6.  Eßbare Folie nach einem der Ansprüche 1 bis 5, wobei die veresterte Stärke mit hohem Amylosegehalt acetylierte Maisstärke mit hohem Amylosegehalt ist.

7.  Eßbare Folie nach einem der Ansprüche 1 bis 6, wobei das Oxidationsprodukt von veretherter Stärke ein Oxidationsprodukt von hydroxypropylierter Stärke ist.

8.  Eßbare Folie nach einem der Ansprüche 1 bis 7, wobei das Säurezersetzungsprodukt von veretherter Stärke ein Säurezersetzungsprodukt von hydroxypropylierter Stärke ist.

9.  Eßbare Folie nach einem der Ansprüche 1 bis 8, wobei das enzymatische Zersetzungspordukt von veretherter Stärke ein Amylasezersetzungsprodukt von hydroxypropylierter Stärke ist.

10. Eßbare Folie nach einem der Ansprüche 1 bis 9, wobei die Folie eine Dicke von 20 bis 200 $\mu$m aufweist.

**Revendications**

1.  Film comestible comprenant :

    (A) un mélange d'amidons modifiés de (a) au moins un amidon modifié choisi dans le groupe constitué par les amidons d'amylose fortement éthérifiés et les amidons d'amylose fortement estérifiés, et (b) au moins un amidon modifié choisi dans le groupe constitué par les produits d'oxydation d'amidon éthérifié, les produits de décomposition acide d'amidon éthérifié, et les produits de décomposition enzymatique d'amidon éthérifié, dans lequel la proportion de (a) / (b) dans le mélange d'amidons modifiés (A) est dans la plage de (a) 30 % à 90 % en poids/ (b) 70 % à 10 % en poids, par rapport au poids total de (a) et (b) ;
    (B) au moins un agent gélifiant ; et
    (C) au moins un plastifiant.

2.  Film comestible selon la revendication 1, dans lequel la proportion de (a)/(b) dans le mélange d'amidons modifiés (A) est dans la plage de (a) 40 % à 80 % en poids/(b) 60 % à 20 % en poids, par rapport au poids total de (a) et (b)

3.  Film comestible selon la revendication 1 ou 2, dans lequel la proportion d'agent gélifiant (B) est dans la plage de 12 à 50 parties en poids pour 100 parties en poids du mélange d'amidons modifiés (A).

4.  Film comestible selon l'une quelconque des revendications 1 à 3, dans lequel la proportion de plastifiant (C) est dans la plage de 3 à 40 parties en poids pour 100 parties en poids du mélange d'amidons modifiés (A).

5.  Film comestible selon l'une quelconque des revendications 1 à 4, dans lequel l'amidon d'amylose fortement éthérifié est l'amidon de maïs d'amylose fortement hydroxypropylé.

6.  Film comestible selon l'une quelconque des revendications 1 à 5, dans lequel l'amidon d'amylose fortement estérifié est l'amidon de maïs d'amylose fortement acétylé.

7.  Film comestible selon l'une quelconque des revendications 1 à 6, dans lequel le produit d'oxydation d'amidon éthérifié est un produit d'oxydation d'un amidon hydroxypropylé.

8.  Film comestible selon l'une quelconque des revendications 1 à 7, dans lequel le produit de décomposition acide d'amidon éthérifié est un produit de décomposition acide d'un amidon hydroxypropylé.

9.  Film comestible selon l'une quelconque des revendications 1 à 8, dans lequel le produit de décomposition enzymatique d'amidon éthérifié est un produit de décomposition par amylase d'un amidon hydroxypropylé.

10. Film comestible selon l'une quelconque des revendications 1 à 9, dans lequel le film a une épaisseur de 20 à 200 $\mu$m.

**REFERENCES CITED IN THE DESCRIPTION**

**Patent documents cited in the description**

- JP 5236885 A
- US 6656493 B
- US 6528088 B
- US 20030099692 A
- US 3527646 A
- EP 0547551 A
- US 6375981 B

**Non-patent literature cited in the description**

- **JOHNSON.** *Analytical Chemistry,* 1969, vol. 41 (6), 859-860
- **SMITH.** Starch: Chemistry and Technology. Academic Press, 1967, vol. 2, 610-612